(19)

Europäisches
Patentamt
European
Patent Office
Office européen
des brevets

(11)     **EP 1 305 075 B1**

(12)                    **EUROPEAN PATENT SPECIFICATION**

(45) Date of publication and mention
of the grant of the patent:
**10.10.2007   Bulletin 2007/41**

(51) Int Cl.:
***A61M 25/04*** *(2006.01)*

(21) Application number: **01953239.9**

(22) Date of filing: **31.07.2001**

(86) International application number:
**PCT/GB2001/003434**

(87) International publication number:
**WO 2002/011810 (14.02.2002 Gazette 2002/07)**

(54) **BALLOON-FREE URINARY CATHETER**

HARNKATHETER OHNE BALLON

CATHETER URINAIRE SANS BALLONNET

(84) Designated Contracting States:
**AT BE CH CY DE DK ES FI FR GB GR IE IT LI LU
MC NL PT SE TR**

(30) Priority: **03.08.2000   GB 0019107**

(43) Date of publication of application:
**02.05.2003   Bulletin 2003/18**

(73) Proprietor: **Ranier Limited
Cambridge CB5 8AA (GB)**

(72) Inventor: **SNELL, Robert Adam
Newmarket,
Suffolk CB8 8AA (GB)**

(74) Representative: **Marsh, Roy David et al
Hoffmann Eitle,
Patent- und Rechtsanwälte,
Arabellastrasse 4
81925 München (DE)**

(56) References cited:
| | |
|---|---|
| EP-A- 0 622 059 | WO-A-96/02214 |
| US-A- 3 344 791 | US-A- 3 616 799 |
| US-A- 5 041 093 | US-A- 5 061 275 |
| US-A- 5 352 182 | US-A- 5 749 826 |
| US-A- 6 053 903 | US-A- 6 096 013 |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

This invention relates to a balloon-free urinary catheter.

Many people suffer from a variety of problems with their urinary system ranging from the inability or difficulty to pass urine to urinary incontinence which can be intermittent or chronic. The most common treatment for many of these disorders is to fit a FOLEY catheter to control drainage of urine from the bladder. A FOLEY catheter is a flexible tube, usually made from latex, which has a central lumen and an inflatable balloon incorporated into a wall of its distal end. With the balloon deflated, the catheter tube can be passed through the urethra into the bladder. Once the balloon at the distal end of the tube is located in the bladder, it is inflated and then rests in the neck of the bladder to provide a seal therewith and prevent the tube from being easily removed. A tubular extension extends a few centimetres beyond the top of the balloon and has holes in its wall to allow urine to pass from the bladder into the central lumen tube. It then flows to the proximal end of the device which is fitted with a connector to inflate the balloon and a second connector to close the central lumen. A collection bag is usually connected to the second connector.

The FOLEY catheter was developed over 40 years ago and has many disadvantages. Firstly, about half of all catheterised patients block their catheters. The FOLEY catheter does not allow for easy removal and cleaning or back-flushing and drainage whilst inserted. Furthermore, it does not deal effectively with those patients whose catheters block and most catheterised patients develop infections of some sort when using a FOLEY catheter which in some cases can be problematic. The FOLEY catheter is very obtrusive, particularly for elderly patients requiring long term catheterisation (the largest group) so its use represents a significant degradation in their quality of life. The major advantage of the FOLEY design is that it is relatively cheap to manufacture, although it does require multiple-dip coatings of latex which can be time consuming. It is also widely available and accepted by the medical profession despite its shortcomings.

U.S. Patent 3,344,791, in an effort to overcome the problems associated with the Foley-type catheter, discloses a catheter having a rubber head having small openings, which, in the relaxed state, forms a bulbous head.

U.S. Patent 5,749,826, in a similar effort, discloses a catheter having outwardly biased flanges made of latex, silicon rubber, or other similar material which expand to engage the bladder.

U.S. Patent 6,053,903 discloses the use of a stiffener ribbon, typically metallic, wound within a catheter body so as to create a catheter having a controllable stiffness. This document further discusses, in a summary of the background art, the use of a braided band as a reinforcement material.

EP 0 622 059 A1 discloses a metallic braided type prostatic stent having a length sufficient to extend from a bladder neck to a position somewhat short of the external sphincter. This device has been designed to non-surgically treat the obstruction of the prostatic urethra caused by the enlargement of the prostatic gland.

It is an object of the present invention to provide a urinary catheter which overcomes or substantially reduces the aforementioned disadvantages.

Summary of the Invention

The invention is defined by the independent claim, the preamble of which is based on U.S. Patent 5,749,826. The dependent claims define preferred embodiments.

According to the present invention, there is provided a balloon-free urinary catheter comprising a tubular retention section which, in use, locates in the neck of a patient's bladder and a tubular drainage section which, in use, is received in the patient's urethra, the retention section having a diameter which is normally greater than the neck of the bladder but being stretchable on the application of an axial force thereto to reduce its diameter to less than that of the urethra to allow the catheter to travel therethrough until the retention section is positioned inside the bladder, the retention section being constructed so that it will expand and return to its original size on release of said axial force whereby it locates and engages with the neck of the bladder to form a seal.

The retention section and drainage section are made of braided material.

The retention section is of a greater diameter than the drainage section and incorporates locating means in the distal end thereof against which said axial force can be applied using a placement tool to axially stretch said section and thereby reduce its diameter. The locating means can be made of any suitable hard polymer material such as nylon, polypropylene, polyethylene, polycarbonate, PTFE or a shape memory metal such as NITINOL.

Spring means can be incorporated into the retention section to assist in its recovery to its original diameter on release of the axial force. Preferably, the spring means are made of a polymer material such as polypropylene, nylon, polyurethane or polyethylene.

In the preferred embodiment, the tubular retention section in its normal relaxed or rest condition has an internal diameter in the range of 12-30 mm, the outer diameter of the tubular drainage section being in the range from 5-12 mm.

The retention section is made of a braided material including structural filaments that are arranged at an angle such that when the axial force is applied to the retention section, the tube decreases in diameter but returns to its original shape due to the hoop stress within the braid structure. Preferably, the retention section is made of a polymer such as polyurethane, neoprene, styrene ethylene butadiene styrene (SEBS), styrene butadiene styrene (SBS), plasticised PVC or thermoplastic valcanites. The structural filaments can be made of a ceramic material or metallic wires or polypropylene, nylon, polyurethane, polyethylene.

The diameter of the retention section increases on the removal of said axial force until the angle of the braids in relation to the longitudinal axis of the catheter reaches a critical angle. The preferred critical angle is $\tan^{-1}\sqrt{2} = 54.73°$.

The tubular drainage section is also made of a braided material including structural filaments that are arranged at an angle such that when the axial force is applied thereto, the tube decreases in diameter but returns to its original shape due to the hoop stress within the braid structure.

In the preferred embodiment, the braid angle of the filaments in the tubular retention and drainage sections is different so that said tubular sections will expand differentially on release of the axial pressure applied to the retention section.

Preferably the catheter is made of a thin walled elastomeric polymer tubing for example polyurethane, neoprene, styrene ethylene butadiene styrene (SEBS), styrene butadiene styrene (SBS), plasticised PVC or thermoplastic vulcanites.

Conveniently a valve is located in the proximal end of the catheter which can be of any convenient type.

Description of the Drawings

Embodiments of the invention will now be described, by way of example only, with reference to the accompanying drawings, in which

Figure 1 is a cross section of the area surrounding the urinary tract showing a prior art FOLEY catheter fitted in the bladder;
Figure 2 is a side view of a urinary catheter of the present invention in its normal relaxed state;
Figure 3 is a view of the catheter shown in Figure 2 in its elongated reduced diameter state ready for insertion into the bladder;
Figure 4 shows the urinary catheter of Figures 2 and 3 but in its relaxed normal state when in position in a patient's bladder;
Figure 5 is an end view of the catheter shown in Figures 2-4;
Figure 6 is a cross section through the catheter of Figures 2-5 but including the placement tool;
Figure 7 is an end view of the catheter of Figures 2-6 but including a return spring in the retention section; and
Figure 8 shows the catheter of Figure 4 in position in a patient's bladder.

Detailed Description

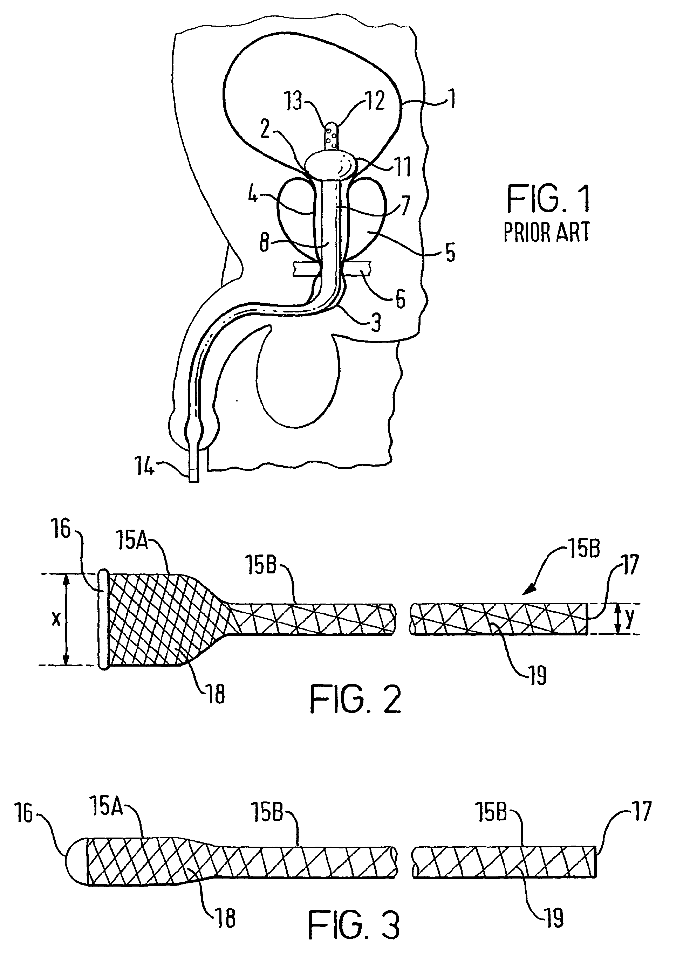

Referring to Figure 1 of the drawings, there is shown a bladder 1 having a neck 2 which tapers into prostatic part 4 of urethra 3 which is surrounded by prostate gland 5. The external sphincter 6 is also shown whose normal function is to keep the urethra closed until urination when it opens for emptying the bladder 1.

A prior art FOLEY type catheter 7 having an elongate body section 8 and a balloon 11 at one end is seated in the tapered neck 2 of the bladder 1 to make a seal therewith. The balloon 11 has a tubular section 12 extending therefrom with holes 13 therein.

To insert the catheter 7 into the bladder 1, the balloon 11 is collapsed and the catheter 7 is inserted through the urethra 3 until the balloon portion 11 has travelled beyond the tapered neck 2 of the bladder 1. The balloon 11 is then inflated to its condition illustrated in Figure 1 as a result of which it makes a seal with the tapered neck 2 of the bladder 1. The body portion 8 of the catheter 7 is located within the prostatic part 4 of the urethra 3 and its distal end 14 protrudes just beyond the end of the urethra 3. A valve incorporating a connector (not shown) would normally be attached to the distal end 14. Urine drains from the bladder 1 through the holes 13 in the extension 12 of the balloon 11 and passes out of the distal end 14 of the catheter to a bag (not shown) connected to the connector in said distal end.

Referring now to the catheter 15 of the present invention shown in Figures 2-6, it can be seen that it comprises a tubular retention section 15A connected to a tubular drainage section 15B of reduced diameter. The retention section 15A has a normal outside diameter "X" in the range of 12-30mm and the drainage section 15B has a normal outside diameter "Y" of between 5-12mm. The length of the catheter is normally in the range of 10-50cms. These dimensions are preferred rather than essential.

A flexible bead 16 is incorporated into the periphery of the proximal end of the retention section 15A for reasons to be explained hereafter. Fingers 16A (four are illustrated but any number could be used) extend radially inwardly to support locating means 20 having a central hole 21 therein. The catheter 15 is made from thin walled elastomeric polymer tubing which incorporates an expandable, braid-activated retainer. Preferred materials for the tubing are polyurethane, neoprene, styrene ethylene butadiene styrene (SEBS), styrene butadiene styrene (SBS), plasticised PVC or thermoplastic vulcanates. The structural filaments of the braiding are arranged at such an angle that when a longitudinal tensioning load is applied to the tube 15 in the direction of arrow A (for instance as shown in Figure 6), it elongates but also decreases in diameter as shown in Figure 3. However, when the tensioning load is released, the hoop stress within the braid structure dictates that the diameter of the tube 15 will increase until the angle of the braid, in relation to the axis of the tube, reaches a critical angle which can be defined as $\tan^{-1}\sqrt{42} = 54.73°$. As can be seen from Figure 2, the braid angle of the retention section 15A is different from that of the drainage section 15B. As a result, when the tube is released from tension as will be described shortly, sections 15A and 15B will expand differentially. This provides a tube that has different diameters relating to the diameter at which the braided component reaches its critical angle.

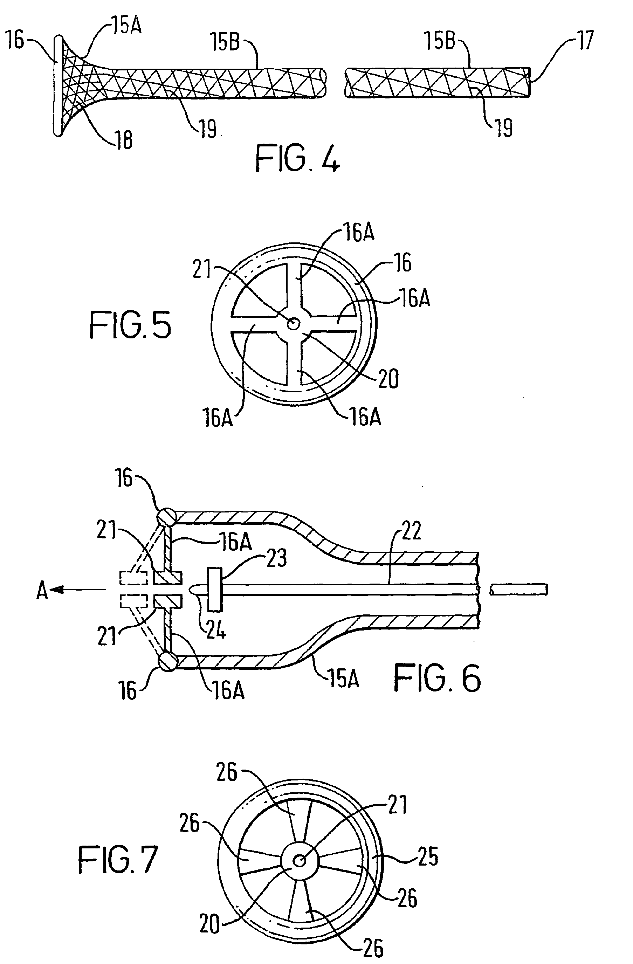

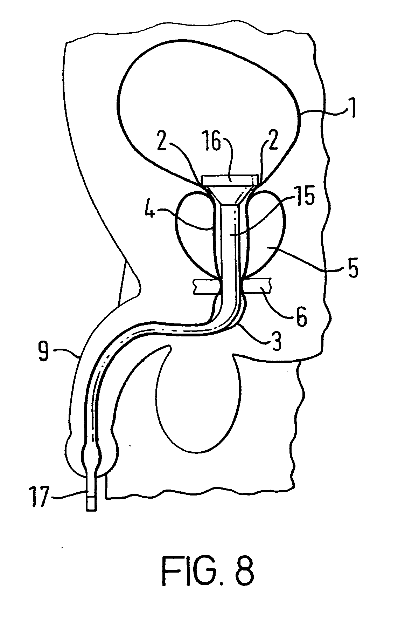

As already explained, catheter 15 shown in Figure 2 is in its normal relaxed condition in which the outer diameter "X" of the retention section 15A is in the range from 12-30mm whereas the outer diameter "Y" of the drainage section 15B is in the range of 5-12mm. To insert the catheter 15 into a patient's bladder, a placement tool (known as a boogie) is used. The tool 22 is inserted into the distal end 17 of the catheter 15 and it is pushed along it until the nose 24 seats in the hole 21 in the locating means 20. Further axial force applied to the boogie in the direction of arrow A causes the retention section 15A and, if desired, the drainage section 15B, to reduce in diameter to approximately 5mm thereby facilitating easy placement of the catheter in the urinary duct 3. As can be seen from Figure 6, the end of the catheter 15 adopts the configuration shown in dotted line during insertion of the catheter. Once the catheter is in place with its retention section 15A in the tapered bladder neck 2, the longitudinal tensioning force applied by the insertion tool 22 can be released thereby allowing the retaining section 15A to expand back to its normal configuration shown in Figure 1 assisted by the resilient bead 16. Because full expansion is constricted by the shape and dimensions of the tapered bladder neck 2, it in fact assumes the configuration shown in Figure 4 when it is properly in position as shown in Figure 8.

The catheter of the invention is made of a bio-compatible polymer material which reduces the rate of bio-film build up and reduce the levels of encrustation and infection normally associated with traditional devices. It also has a thin wall compared to prior art catheters which is achieved due to the high hoop strength of the braided tubing allowing a braided tube having a wall thickness of approximately 100 microns with an outer diameter x which performs with the same kink resistance as a latex tube with an outer diameter of x and a 2mm wall thickness. This enables the lumen size in the catheter of the invention to be significantly larger within the braided tube 15 compared to prior art latex catheters thereby facilitating higher flow rates within the catheter.

Prototype devices have been evaluated using an in vitro artificial bladder model (Stickler D J, Morris N S and Winters C 'Simple physical model to study formation and physiology of biofilms on urethral catheters' Methods in Enzymology 310, p494-501, 1999; Stickler D J, Howe N S and Winters C, 'Bacterial film growth on ciprofloxacin treated urethral catheters' Cells Materials 4, p245-253, 1994) to determine the efficacy of catheter designs to provide functional drainage under adverse conditions in which artificial urine is inoculated with bacteria to induce urease production that initiates the cascade (Bruce A.W. Sira S.S., Clark A.F. and Awad S.A. The problem of catheter encrustation. Can Med Ass J 111: p238-241, 1974; Stickler D J, Morris N S, Moreno M and Sabbuba N. Studies on the formation of crystalline bacterial biofilms on urethral catheters. Eur J Microbiol Infect Dis., 17: p1-4, 1998; Brocklehurst J C and Brocklehurst, S. The management of indwelling catheters. Br.J Urol 50: p102-105, 1978; Warren J W, Platt R. Thomas R J, Rosner B, and Kass E H, 'Antibiotic Irrigation and Catheter-associated Urinary Tract Infections'. New England J Med 299: p570-573, 1978) resulting in precipitation of calcium and magnesium phosphate salts that subsequently block urethral catheters. Table 1 shows how the preferred catheter of the invention exceeded the performance of conventional urinary catheter designs (Morris N S, Stickler D B, and Winters C 'Which indwelling urethral catheters resist encrustation by Proteus mirabilis biofilms? Br J Urol 80, p58-63, 1997) thus providing an effective demonstration of the benefits of the design.

**TABLE 1**

| Catheter blockage times in the bladder model inoculated with *Proteus mirabilis* | | | |
|---|---|---|---|
| **Design** | **Material** | **Coated (treated)** | **Time (hr)** |
| Foley | Latex | Hyrdrogel (silver) | 21 |
| Foley | Latex | Hydrogel | 22 |
| Foley | Latex | (silicone) | 23 |
| Foley | Latex | Teflon | 27 |
| Foley | Silicone | na | 44 |
| Foley | PVC | - | 48 |
| Foley | Silicone | na | 52 |

(continued)

| Catheter blockage times in the bladder model inoculated with *Proteus mirabilis* | | | |
|---|---|---|---|
| **Design** | **Material** | **Coated (treated)** | **Time (hr)** |
| Balloon free design of the invention | Polyurethane | na | > 144 |
| na: not applicable | | | |

Polyurethane material which soften at body temperature can be used to give a more conformant and comfortable fit for the patient without the concern of latex allergy. Materials that soften significantly on placement can also be used to hold the retaining section in its low diameter configuration during handling so that when the material softens, the retention section 15A of the catheter can be deployed.

It is also envisaged within the scope of the invention that a polymeric spring 25 such as that shown in Figure 7 could be incorporated into the retention section or funnel end 15A to facilitate funnel opening and ease of placement and to modify the retention force. This comprises fingers 26 (four are shown but any number could be used) connected to central locating means 20 having a central hole 21.

The catheter of the present invention has the advantage that it provides control over the design parameters which allows for a range of performance criteria to be satisfied. The variables, which are easily changed, are the polymeric material, which affect the surface properties of the device and contribute to the physical properties of the tubing. The braid material can also be altered in shape and size to give a range of properties and also in configuration to allow modification of the three dimensional relaxed and stressed states of the tube. The manufacturing method of the present invention also allows the placement of inlaid wires or fibres within the drainage section 15B to transfer the longitudinal tension away from the braided section 15A thus preventing elongation and diameter changes within the drainage section 15B.

A further advantage of the braided catheter of the present invention is it can be manufactured by a cost effective continuous co-extrusion process thereby avoiding the time consuming multiple-dip costing processes of prior art catheters.

## Claims

1. A balloon-free urinary catheter comprising a tubular retention section (15A) which, in use, locates in the neck of a patient's bladder and a tubular drainage section (15B) which, in use, is received in the patient's urethra, the retention section having a diameter which is normally greater than the neck of the bladder but being stretchable on the application of an axial force thereto to reduce its diameter to less than that of the urethra to allow the catheter to travel therethrough until the retention section is positioned inside the bladder, the retention section (15A) being constructed so that it will expand and return to its original size on release of said axial force whereby it locates in the neck of the bladder, whereby the retention section (15A) engages with the neck of the bladder to form a seal, **characterised in that** the retention section (15A) and the drainage section (15B) are both made of a braided material comprising structural filaments arranged at a braid angle so that, when said axial force is applied to the catheter, the retention section (15A) and the drainage section (15B) decrease in diameter and once the axial force is removed, the diameter of the tubular retention section is at its greatest at its end remote from the drainage section, so as to exhibit a funnel shape.

2. A urinary catheter as claimed in claim 1, wherein the braid angle of the retention section (15A) and the drainage section (15B) is different so that the tubular sections will expand differentially on release of the axial force applied to the retention section.

3. A urinary catheter as claimed in claim 1 or claim 2 **characterised in that** the retention section (15A) is of a greater diameter than the drainage section (15B).

4. A urinary catheter as claimed in any preceding claim **characterised in that** locating means (16) are incorporated into the distal end of the retention section (15A) against which said axial force can be applied to stretch said section and reduce its diameter.

5. A urinary catheter as claimed in claim 4 **characterised in that** said locating means (16) is made of a shape memory metal.

6. A urinary catheter as claimed in claim 4 **characterised in that** the locating means is made of a polymeric material such as nylon, polypropylene, polyethylene, polycarbonate or PTFE.

7. A urinary catheter as claimed in any preceding claim **characterised in that** spring means incorporated into the retention section (15A) to assist in its recovery to its original diameter on release of the axial force.

8. A urinary catheter as claimed in claim 7 **characterised in that** the spring means is radially expandable.

9. A urinary catheter as claimed in claim 8 **characterised in that** the spring means is made of a polymer material.

10. A urinary catheter as claimed in claim 9 **characterised in that** the spring means is made of polypropylene, nylon, polyurethane or polyethylene.

11. A urinary catheter as claimed in any preceding claim **characterised in that** a flexible bead (16) is provided around the distal end of the retention section (15A) to restore said section to its original shape on release of said axial force.

12. A urinary catheter as claimed in claim 11 **characterised in that** the bead (16) is made of an elastomeric polymer.

13. A urinary catheter as claimed in any preceding claim **characterised in that** the outer diameter of the tubular retention section (15A) in its relaxed condition is in the range from 12-30mm.

14. A urinary catheter as claimed in any preceding claim **characterised in that** the outer diameter of the tubular drainage section (15B) is in the range from 5-12mm.

15. A urinary catheter as claimed in any preceding claim, **characterised in that** the diameter of the retention tube increases on the removal of the axial force until the braid angle in relation to the longitudinal axis of the catheter reaches a critical angle.

16. A urinary catheter as claimed in claim 15, **characterised in that** the critical angle is $\tan^{-1}\sqrt{2} = 54.73°$.

17. A urinary catheter as claimed in any preceding claim **characterised in that** the catheter is made of thin walled polyurethane tubing.

18. A urinary catheter as claimed in any preceding claim **characterised in that** a valve is located in the proximal end of the catheter.


**Patentansprüche**

1. Ballonfreier Harnblasenkatheter mit einem schlauchförmigen Halteabschnitt (15A), der im Gebrauch in den Blasenhals des Patienten gelegt wird, und einem schlauchförmigen Entleerungsabschnitt (15B), der im Gebrauch in der Harnröhre des Patienten aufgenommen wird, wobei der Halteabschnitt einen Durchmesser besitzt, der normalerweise größer als der Blasenhals ist, jedoch bei Anwenden einer Axialkraft auf ihn dehnbar ist, um seinen Durchmesser auf weniger als jenen der Harnröhre zu verringern und es so dem Katheter zu erlauben, durch sie zu verfahren bis der Halteabschnitt in der Blase positioniert ist, wobei der Halteabschnitt (15A) so konstruiert ist, dass er sich ausdehnen und zu seiner ursprünglichen Größe zurückkehren wird, wenn die Axialkraft gelockert wird, wodurch er im Blasenhals örtlich festgelegt wird, wobei der Halteabschnitt (15A) mit dem Blasenhals so eingreift, dass er eine Abdichtung bildet,
**dadurch gekennzeichnet, dass** der Halteabschnitt (15A) und der Entleerungsabschnitt (15B) beide aus einem geflochtenen Material hergestellt sind, das strukturelle Filamente umfasst, die unter einem Geflechtwinkel angeordnet sind, so dass bei Anwenden der Axialkraft auf den Katheter der Halteabschnitt (15A) und der Entleerungsabschnitt (15B) im Durchmesser abnehmen und,
sobald die Axialkraft gelockert wird, der Durchmesser des schlauchförmigen Halteabschnitts an seinem vom Entleerungsabschnitt entfernten Ende am größten ist, um so eine Trichterform aufzuweisen.

2. Harnblasenkatheter nach Anspruch 1, wobei der Geflechtwinkel des Halteabschnitts (15A) und des Entleerungsabschnitts (15B) unterschiedlich ist, so dass die rohrförmigen Abschnitte sich beim Lockern der auf den Halteabschnitt ausgeübten Axialkraft unterschiedlich ausdehnen werden.

3. Harnblasenkatheter nach Anspruch 1 oder Anspruch 2, **dadurch gekennzeichnet, dass** der Halteabschnitt (15A) einen größeren Durchmesser besitzt als der Entleerungsabschnitt (15B).

4. Harnblasenkatheter nach einem der vorangehenden Ansprüche, **dadurch gekennzeichnet, dass** Lokalisierungsmittel (16) in das distale Ende des Halteabschnitts (15A) eingearbeitet sind, gegen die die Axialkraft ausgeübt werden kann, um den Abschnitt auszudehnen und seinen Durchmesser zu verringern.

5. Harnblasenkatheter nach Anspruch 4, **dadurch gekennzeichnet, dass** das Lokalisierungsmittel (16) aus einem Formgedächtnismetall hergestellt ist.

6. Harnblasenkatheter nach Anspruch 4, **dadurch gekennzeichnet, dass** das Lokalisierungsmittel aus einem Polymermaterial wie z.B. Nylon, Polypropylen, Polyethylen, Polycarbonat oder PTFE besteht.

7. Harnblasenkatheter nach einem der vorangehenden Ansprüche, **dadurch gekennzeichnet, dass** ein Federmittel in den Halteabschnitt (15A) eingearbeitet ist, um beim Lockern der Axialkraft bei der Wiedererlangung seines ursprünglichen Durchmessers zu helfen.

8. Harnblasenkatheter nach Anspruch 7, **dadurch gekennzeichnet, dass** das Federmittel radial expandierbar ist.

9. Harnblasenkatheter nach Anspruch 8, **dadurch gekennzeichnet, dass** das Federmittel aus einem Polymermaterial besteht.

10. Harnblasenkatheter nach Anspruch 9, **dadurch gekennzeichnet, dass** das Federmittel aus Polypropylen, Nylon, Polyurethan oder Polyethylen besteht.

11. Harnblasenkatheter nach einem der vorangehenden Ansprüche, **dadurch gekennzeichnet, dass** eine flexible Wulst (16) um das distale Ende des Halteabschnitts (15A) herum vorgesehen ist, um den Abschnitt beim Lockern der Axialkraft zu seiner ursprünglichen Form wiederherzustellen.

12. Harnblasenkatheter nach Anspruch 11, **dadurch gekennzeichnet, dass** der Wulst (16) aus einem elastomerischen Polymer besteht.

13. Harnblasenkatheter nach einem der vorangehenden Ansprüche, **dadurch gekennzeichnet, dass** der äußere Durchmesser des schlauchförmigen Halteabschnitts (15A) in seinem entspannten Zustand im Bereich von 12 bis 30 mm liegt.

14. Harnblasenkatheter nach einem der vorangehenden Ansprüche, **dadurch gekennzeichnet, dass** der äußere Durchmesser des schlauchförmigen Entleerungsabschnitts (15B) im Bereich von 5 bis 12 mm liegt.

15. Harnblasenkatheter nach einem der vorangehenden Ansprüche, **dadurch gekennzeichnet, dass** der Durchmesser des Halteschlauchs bei Entfernen der Axialkraft zunimmt, bis der Geflechtwinkel in Bezug auf die Längsachse des Katheters einen kritischen Winkel erreicht.

16. Harnblasenkatheter nach Anspruch 15, **dadurch gekennzeichnet, dass** der kritische Winkel $\tan^{-1}\sqrt{2} = 54.73°$ beträgt.

17. Harnblasenkatheter nach einem der vorangehenden Ansprüche, **dadurch gekennzeichnet, dass** der Katheter aus einem dünnwandigen Polyurethanschlauch besteht.

18. Harnblasenkatheter nach einem der vorangehenden Ansprüche, **dadurch gekennzeichnet, dass** am proximalen Ende des Katheters ein Ventil platziert ist.

**Revendications**

1. Cathéter urinaire sans ballon comprenant une section de rétention tubulaire (15A) qui, à l'usage, se situe dans le col de la vessie d'un patient et une section de drainage tubulaire (15B) qui, à l'usage, est reçue dans l'urètre du patient, la section de rétention ayant un diamètre qui est normalement supérieur au col de la vessie mais étant étirable à l'application d'une force axiale sur lui pour réduire son diamètre à moins que celui de l'urètre pour permettre au cathéter de se déplacer à travers celui-ci jusqu'à ce que la section de rétention soit positionnée à l'intérieur de la vessie, la section de rétention(15A) étant construite de sorte qu'elle se dilatera et retournera à sa taille originale au relâchement de ladite force axiale au moyen de quoi elle se situe dans le col de la vessie, au moyen de quoi la section de rétention (15A) s'engage avec le col de la vessie pour former un joint,

**caractérisé en ce que** la section de rétention (15A) et la section de drainage (15B) sont faites toutes deux d'un matériau tressé comprenant des filaments de structure agencés au niveau d'un angle de tressage de sorte que, lorsque ladite force axiale est appliquée au cathéter, le diamètre de la section de rétention (15A) et de la section de drainage (15B) diminue, et

une fois que la force axiale est retirée, le diamètre de la section de rétention tubulaire est à son maximum au niveau de son extrémité distante de la section de drainage, de sorte à présenter une forme d'entonnoir.

2. Cathéter urinaire tel que revendiqué dans la revendication 1, dans lequel l'angle de tressage de la section de rétention (15A) et la section de drainage (15B) est différent de sorte que les sections tubulaires se dilateront différemment au relâchement de la force axiale appliquée à la section de rétention.

3. Cathéter urinaire tel que revendiqué dans la revendication 1 ou la revendication 2, **caractérisé en ce que** la section de rétention (15A) est d'un diamètre supérieur à celui de la section de drainage (15B).

4. Cathéter urinaire tel que revendiqué dans l'une quelconque des revendications précédentes, **caractérisé en ce que** des moyens de localisation (16) sont incorporés dans l'extrémité distale de la section de rétention (15A) contre lesquels ladite force axiale peut être appliquée pour étirer ladite section et réduire son diamètre.

5. Cathéter urinaire tel que revendiqué dans la revendication 4, **caractérisé en ce que** ledit moyen de localisation (16) est fait d'un métal à mémoire de forme.

6. Cathéter urinaire tel que revendiqué dans la revendication 4, **caractérisé en ce que** le moyen de localisation (16) est fait d'un matériau polymère tel que le nylon, le polypropylène, le polyéthylène, le polycarbonate ou le PTFE.

7. Cathéter urinaire tel que revendiqué dans l'une quelconque des revendications précédentes, **caractérisé en ce que** un moyen de ressort est incorporé dans la section de rétention (15A) pour aider à son retour à son diamètre original au relâchement de la force axiale.

8. Cathéter urinaire tel que revendiqué dans la revendication 7, **caractérisé en ce que** le moyen de ressort peut être dilaté radialement.

9. Cathéter urinaire tel que revendiqué dans la revendication 8, **caractérisé en ce que** le moyen de ressort est fait d'un matériau polymère.

10. Cathéter urinaire tel que revendiqué dans la revendication 9, **caractérisé en ce que** le moyen de ressort est fait de polypropylène, de nylon, de polyuréthane ou de polyéthylène

11. Cathéter urinaire tel que revendiqué dans l'une quelconque des revendications précédentes, **caractérisé en ce que** une bille flexible (16) est prévue autour de l'extrémité distale de la section de rétention (15A) pour restaurer ladite section à sa forme originale au relâchement de ladite force axiale.

12. Cathéter urinaire tel que revendiqué dans la revendication 11, **caractérisé en ce que** la bille (16) est faite d'un polymère élastomère.

13. Cathéter urinaire tel que revendiqué dans l'une quelconque des revendications précédentes, **caractérisé en ce que** le diamètre externe de la section de rétention tubulaire (15A) à l'état relâché se situe dans la gamme de 12 à 30 mm.

14. Cathéter urinaire tel que revendiqué dans l'une quelconque des revendications précédentes, **caractérisé en ce que** le diamètre externe de la section de drainage tubulaire (15B) se situe dans la gamme de 5 à 12 mm.

15. Cathéter urinaire tel que revendiqué dans l'une quelconque des revendications précédentes, **caractérisé en ce**

**que** le diamètre du tube de rétention augmente au retrait de la force axiale jusqu'à ce que l'angle de tressage par rapport à l'axe longitudinal du cathéter atteigne un angle critique.

16. Cathéter urinaire tel que revendiqué dans la revendication 15, **caractérisé en ce que** l'angle critique est $\tan^{-1}\sqrt{2}$ =54, 53°.

17. Cathéter urinaire tel que revendiqué dans l'une quelconque des revendications précédentes, **caractérisé en ce que** le cathéter est fait d'un tube de polyuréthane à paroi mince.

18. Cathéter urinaire tel que revendiqué dans l'une quelconque des revendications précédentes, **caractérisé en ce que** une valve est située dans l'extrémité proximale du cathéter.

FIG. 1
PRIOR ART

FIG. 2

FIG. 3

FIG. 4

FIG.5

FIG. 6

FIG.7

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 3344791 A
- US 5749826 A
- US 6053903 A
- EP 0622059 A1

### Non-patent literature cited in the description

- **STICKLER D J ; MORRIS N S ; WINTERS C.** Simple physical model to study formation and physiology of biofilms on urethral catheters. *Methods in Enzymology,* 1999, vol. 310, 494-501
- **STICKLER D J ; HOWE N S ; WINTERS C.** Bacterial film growth on ciprofloxacin treated urethral catheters. *Cells Materials,* 1994, vol. 4, 245-253
- **BRUCE A.W. ; SIRA S.S. ; CLARK A.F. ; AWAD S.A.** The problem of catheter encrustation. *Can Med Ass J,* 1974, vol. 111, 238-241
- **STICKLER D J ; MORRIS N S ; MORENO M ; SAB-BUBA N.** Studies on the formation of crystalline bacterial biofilms on urethral catheters. *Eur J Microbiol Infect Dis.,* 1998, vol. 17, 1-4
- **BROCKLEHURST J C ; BROCKLEHURST, S.** The management of indwelling catheters. *Br.J Urol,* 1978, vol. 50, 102-105
- **WARREN J W ; PLATT R. ; THOMAS R J ; ROSNER B ; KASS E H.** Antibiotic Irrigation and Catheter-associated Urinary Tract Infections. *New England J Med,* 1978, vol. 299, 570-573
- **MORRIS N S ; STICKLER D B ; WINTERS C.** Which indwelling urethral catheters resist encrustation by Proteus mirabilis biofilms?. *Br J Urol,* 1997, vol. 80, 58-63